# EUROPEAN PATENT APPLICATION

(11) **EP 4 722 712 A1**
(43) Date of publication of application: **08.04.2026**
(21) Application number: 24811131.2
(22) Date of filing: 22.05.2024
(51) Int. Cl.: G01N 30/88, G01N 30/86, G01N 33/72

(54) **METHOD FOR ANALYZING HEMOGLOBIN AND SYSTEM FOR ANALYZING HEMOGLOBIN**

(30) Priority: 24.05.2023 JP 2023085211
(71) Applicant: Tosoh Corporation, Yamaguchi 746-8501 (JP)
(72) Inventor: MANITA, Daisuke, Ayase-shi, Kanagawa 252-1123 (JP); OGINO, Shinji, Shunan-shi, Yamaguchi 746-8501 (JP)
(74) Representative: Novagraaf International SA
(86) International application number: PCT/JP2024/018767
(87) International publication number: WO 2024/242125

(57) **Abstract**

The present invention provides a method for analyzing hemoglobin in a blood-derived sample and a system for implementing said method, said method comprising: (1) a step for identifying, from a chromatogram that is obtained by subjecting a blood-derived sample to cation exchange liquid chromatography and that represents the elution of a plurality of hemoglobin fractions which include at least HbAlc and HbA0, a first peak for HbAlc and another peak (second peak) at which elution is faster than that at the first peak; (2) a step for calculating first peak information pertaining to the first peak and second peak information pertaining to the second peak; and (3) a step for deducing the presence of abnormal hemoglobin in the sample from a comparison between the ratio between the first peak information and the second peak information and a prescribed reference threshold value range.

## Description

### FIELD

The present invention relates to a method for analyzing hemoglobin and to a system for analyzing hemoglobin. More specifically, the invention relates to a method and system for measuring HbA1 in a blood sample, which allow estimation of an abnormal hemoglobin-containing specimen by cation-exchange liquid chromatography.

### BACKGROUND

Hemoglobin A1c (HbA1c, or stable HbA1c) is a biomarker that nonenzymatically binds with glucose in the blood (blood glucose) or its metabolites, and it is widely used for diabetes diagnosis and treatment monitoring. Labile HbAlc (LA1c), on the other hand, is in a state with glucose temporarily bonded to hemoglobin A, and normally it is not included in the term "stable HbA1c".

Measurement of HbA1c is widely carried out based on the principle of cation-exchange liquid chromatography (CEX-HPLC). In a hemoglobin fraction measured by this method, the separated peaks are the SA1c peak, which is the peak for HbA1c, and peaks for LA1c, hemoglobin A0 (HbA0 or A0) and hemoglobin F (HbF or F) as fetal hemoglobin.

Methods of measuring HbAlc by CEX-HPLC have the advantage of allowing detection of hemoglobin fractions produced by mutations in the hemoglobin gene, collectively known as abnormal hemoglobin, but also have a disadvantage in that the presence of the latter can potentially interfere with precision of the results for HbA1c.

Hemoglobin J-Baltimore (HbJ-Baltimore) is a type of abnormal hemoglobin that is widely distributed mainly in North America, and since reports indicate that it affects HbAlc measurement results, the recommendation is to verify such results by electrophoresis which, however, is a more complicated procedure (NPL 1). It has therefore been desirable to be able to also estimate the presence of hemoglobin J-Baltimore in CEX-HPLC measurement.

PTL 1 discloses a method for calculating stable HbAlc, which takes into account that in cation-exchange chromatography measurement of a blood sample containing hemoglobin E (HbE), as one type of abnormal hemoglobin, the HbE peak is known to appear between stable HbA1c and HbA.

### [CITATION LIST]

### [PATENT LITERATURE]

[PTL 1] Japanese Unexamined Patent Publication No. 2012-215470

### [NON PATENT LITERATURE]

[NPL 1] MA Gargollo et al., Abnormally low HbAlc secondary to hemoglobin J-Baltimore [beta 16(A13) Gly→Asp]. Family study, Endocrinol Nutr. 2010 Feb; 57(2):83-5

### SUMMARY

### [TECHNICAL PROBLEM]

With specimens containing abnormal hemoglobin HbJ-Baltimore it is not possible to obtain accurate measurement results for HbA1c. It is therefore an object of this invention to develop a method or system that allows the presence of HbJ-Baltimore to be estimated when performing measurement by ion-exchange liquid chromatography.

### [SOLUTION TO PROBLEM]

As a result of much research on this problem, the present inventors have completed this invention upon finding that it is possible to estimate the presence of abnormal hemoglobin (for example, hemoglobin J-Baltimore) from a chromatogram of a sample containing multiple hemoglobins including HbAlc and HbA0. Specifically, the invention has the following aspects.

[1] A method for analyzing hemoglobin from a blood-derived sample, the method comprising:
   (1) a step of identifying a first peak for HbAlc and a different peak (second peak) with earlier elution than the first peak, from a chromatogram representing elution of a plurality of hemoglobin fractions including at least HbAlc and HbA0, obtained by supplying a blood-derived sample to cation-exchange liquid chromatography,
   (2) a step of calculating first peak information for the first peak and second peak information for the second peak, wherein the first peak information and the second peak information each consist of the respective peak heights, peak areas or peak area ratios; and
   (3) a step of estimating the presence of abnormal hemoglobin in the sample based on comparison between the first peak information and the second peak information and a predetermined reference threshold range.
[2] The method according to [1] above, wherein the second peak is a peak for hemoglobin F or LA1c.
[3] The method according to [1] or [2] above, wherein in step (3), it is estimated that abnormal hemoglobin is present in the sample when the ratio of the first peak information and the second peak information has deviated from the predetermined reference threshold range.
[4] The method according to [1] or [2] above, wherein in step (3), it is estimated that abnormal hemoglobin is present in the sample when the ratio of the first peak information and the second peak information has deviated from the predetermined reference threshold range, and in the chromatogram, one or more peaks have been detected that elute later than the first peak and are different from the peak for HbA0.
[5] The method according to [3] or [4] above, wherein when it is estimated that the abnormal hemoglobin is present in the sample, one or more steps selected from among the following steps are carried out:
   (a) the response given is that the measurement results for HbAlc for the sample cannot be reported; and
   (b) the response given is the measurement results for HbAlc for the sample, with an associated identifier indicating the presence of the abnormal hemoglobin.
[6] The method according to any one of [1] to [5] above, wherein the abnormal hemoglobin is hemoglobin J-Baltimore.
[7] The method according to any one of [1] to [6] above, wherein
   the second peak is a peak for LA1c,
   in step (2), the first peak information is the area ratio for HbAlc and the second peak information is the area ratio for LA1c, and
   the ratio of the first peak information and the second peak information in step (3) is the ratio of the area ratio for LAlc and the area ratio for HbAlc (LA1c%/SA1c%).
[8] The method according to [7] above, wherein the predetermined reference threshold range is less than 0.50 or less than 0.70.
[9] A hemoglobin analysis system in which the method according to any one of [1] to [8] above is carried out.
[10] The hemoglobin analysis system according to [9] above, which comprises:
   a cation-exchange liquid chromatography apparatus, and
   analyzing means for analyzing chromatogram data obtained by the cation-exchange liquid chromatography apparatus.

### [ADVANTAGEOUS EFFECTS OF INVENTION]

According to the invention it is possible to estimate the presence of abnormal hemoglobin HbJ-Baltimore, and to reduce reporting of inaccurate HbAlc results in the presence of HbJ-Baltimore.

### BRIEF DESCRIPTION OF DRAWINGS

Fig. 1 is a block diagram of a measuring apparatus according to an embodiment of the disclosure.
Fig. 2 is a flow chart showing an example of analysis processing in a measuring apparatus.
Fig. 3 is an example of a common specimen chromatogram (measured in standard short mode) as measured with the measuring apparatus of Fig. 1.
Fig. 4 is an example of an HbJ specimen chromatogram (measured in standard short mode) as measured with the measuring apparatus of Fig. 1.
Fig. 5 is a histogram showing the distribution of the ratio between the area ratio for LAlc and the area ratio for SA1c (LA1c%/SA1c%) in a common specimen.
Fig. 6 is a histogram showing the distribution of the ratio between the area ratio for LAlc and the area ratio for SA1c (LA1c%/SA1c%) in an HbJ specimen.
Fig. 7 is an example of an HbJ specimen chromatogram (measured in standard long mode) as measured with the measuring apparatus of Fig. 1.

### DESCRIPTION OF EMBODIMENTS

The present invention will now be described in greater detail by Examples, with the understanding that the invention is not limited to the Examples. The entirety of the documents cited throughout the present specification may be incorporated herein by reference.

The terms "first", "second", etc. used throughout the present specification are used only to distinguish one element from another, and a first element may be referred to as "second element", or similarly a second element may be referred to as "first element", without deviating from the gist of the invention.

According to one embodiment, the invention provides a method for analyzing hemoglobin from a blood-derived sample, the method comprising:
(1) a step of identifying a peak for HbAlc ("first peak") and a different peak ("second peak") with earlier elution than the first peak, from a chromatogram representing elution of a plurality of hemoglobin fractions including at least HbAlc and HbA0, obtained by supplying a blood-derived sample to cation-exchange liquid chromatography,
(2) a step of calculating first peak information for the first peak and second peak information for the second peak, wherein the first peak information and the second peak information each consist of the respective peak heights, peak areas or peak area ratios; and
(3) a step of estimating the presence of abnormal hemoglobin in the sample based on comparison between the first peak information and the second peak information and a predetermined reference threshold range.

According to another embodiment, the invention provides a hemoglobin analysis system in which the method described above is carried out. The hemoglobin analysis system provided by the invention may comprise analyzing means for analyzing data from a chromatogram acquired by a cation-exchange liquid chromatography apparatus, and for example, it may be a computer in which a control program for carrying out the method is installed, where the computer comprises a processor (such as a CPU or microcomputer). According to another aspect, the invention may be a computer program that causes the analyzing means to carry out the method described above.

The data for the chromatogram acquired from the cation-exchange liquid chromatography apparatus may be introduced into the analyzing means through a network (wired network or wireless network) or a storage medium (for example, a memory device such as a RAM, ROM or flash memory, a fixed disk device such as a hard disk drive, or a portable storage device such as a flexible disk or optical disk).

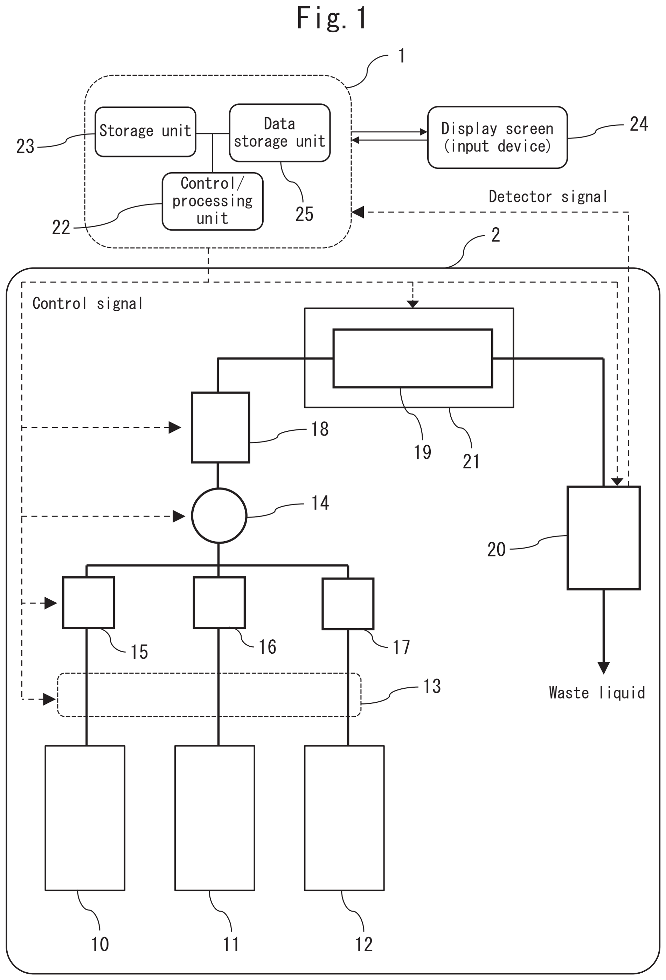

According to another aspect, the invention may comprise a cation-exchange liquid chromatography apparatus and analyzing means for analyzing chromatogram data obtained by the cation-exchange liquid chromatography apparatus (see Fig. 1, for example). In this case, the computer used as the analyzing means may be in a form incorporated into the cation-exchange liquid chromatography apparatus in a non-separable manner, or it may be provided in a so-called "external" form.

The cation-exchange liquid chromatography to be applied for the invention will now be described. A cation-exchange column is prepared from a filler having a cation-exchange group introduced into non-porous crosslinked polymer particles, but the method of synthesizing the non-porous crosslinked polymer particles is not restricted and may be a known suspension polymerization method, emulsion polymerization method, seed polymerization method or precipitation polymerization method. According to the invention, there is no limitation to non-porous crosslinked polymer particles, and a porous crosslinked polymer may be used.

There are no particular restrictions on the monomers and crosslinking agents used for synthesis, but hydrophilic methacrylic acid and acrylic esters are preferred. Monomers include hydroxyethyl methacrylate, hydroxyethyl acrylate, hydroxypropyl acrylate, glycerol methacrylate, polyethylene glycol methacrylate and polyethylene glycol acrylate. Crosslinking agents include ethylene glycol dimethacrylate, diethylene glycol dimethacrylate, triethylene glycol dimethacrylate and glycerol dimethacrylate.

When non-porous crosslinked polymer particles are used, there are no particular restrictions on the method of introducing the cation-exchange group on the surface, and any publicly known introduction method may be used. Cation-exchange groups that may be introduced include sulfopropyl, sulfoethyl and carboxylmethyl groups. The same examples may be mentioned for use of a porous crosslinked polymer.

The HPLC method to be applied for the invention is not restricted, but preferably the one peak from HbJ-Baltimore is caused to elute earlier than the SA1c peak, so as not to affect the HbAlc peak. In addition, since the HbF peak can be used as an index for beta-thalassemia, it is more preferably caused to elute into the LAlc peak, which is one of the earlier elution peaks than the SA1c peak.

Also, a separate HbJ-Baltimore-derived peak is preferably caused to elute later than the SA1c peak, to form a peak that is distinguishable from HbA0.

Abnormal hemoglobin HbJ-Baltimore is hemoglobin having a β-globin chain mutant in which glycine (Gly) at the 16th amino acid of the β-globin chain, as one of the subunits composing hemoglobin, is mutated to aspartic acid (Asp) (see NPL 1, for example).

There are no particular restrictions on the eluent used for cation-exchange liquid chromatography according to the invention, and it may be a buffering solution comprising an organic acid such as succinic acid or citric acid and its salt, a buffering solution comprising an inorganic acid such as phosphoric acid and its salt, or a mixture of both an organic acid and an inorganic acid. If necessary, other salts such as sodium chloride, sodium nitrate or sodium sulfate may be added to the buffering solution.

For the Examples of the present application, the cation-exchange liquid chromatography apparatus used to obtain the chromatogram representing elution of the hemoglobin fraction was an HLC-723GR01 automatic glycohemoglobin analyzer (standard long mode) (Tosoh Corp.). This apparatus is a liquid chromatography apparatus based on the principle of cation-exchange chromatography. The main system configuration of the apparatus will now be explained with reference to Fig. 1.

The analysis column (19) used was TSKgel GR01 (standard short mode and standard long mode) (Tosoh Corp.), and the eluents used were a GR01 eluent first solution (10), a GR01 eluent second solution (11) and a GR01 eluent third solution (12). These measuring conditions allow measurement with a measuring time of 50 seconds per specimen.

Each eluent (10, 11, 12) is conveyed by a liquid feed pump 14 and delivered to an analysis column 19 together with the measuring sample injected by the autosampler 18. Each eluent (10, 11, 12) is passed through a degasser 13 as necessary, creating a different eluent mixing ratio by switching of solenoid valves (15, 16, 17), and the components are separated with an analysis column and the fractions successively eluted, depending on the difference in salt concentration created by the mixing ratio. The successively eluted fractions are guided to a detector 20 and the output value is continuously determined.

The degasser 13, solenoid valves (15, 16, 17), liquid feed pump 14, autosampler 18, column oven 21 and detector 20 are controlled by a control/processing unit 22 which is a microcomputer or CPU, based on the control program and measuring conditions prestored in a storage unit 23. The detector signal obtained by the detector is converted by the control/processing unit 22 into plot data comprising time and a detector signal, for example. This plot data is then used to draw a chromatogram. The chromatogram is further processed by waveform processing and the HbA1c is quantitatively calculated based on previously stored calibration data. The obtained quantitative calculation results and chromatogram may be displayed on a display screen 24, allowing the data to be stored in a data storage unit 25.

According to one aspect of the invention, a chromatogram is acquired based on plot data with time (retention time) on the abscissa and detector output (signal output) such as absorbance on the ordinate, but elution volume may also be plotted on the abscissa. The ordinate may also represent the value calculated from the detector signal. Correction such as noise processing, smoothing, drift processing or baseline compensation, for example, may also be carried out. The method of displaying the chromatogram may be normalization, for comparison while excluding the effects of the measuring conditions and environmental conditions.

Waveform processing is carried out from the chromatogram, and the peaks are identified. For a specimen from a healthy individual, the six peaks for HbA1a, HbA1b, labile HbAlc (LA1c), HbAlc (SA1c) and hemoglobin A0 (HbA0) are separated and identified, in order of more rapid elution. HbA1c is generally calculated from the ratio (percentage) of the area of the SA1c peak with respect to the total hemoglobin area (total area). Calibration is usually carried out based on measurement results for known samples, in order to correct for deviations from true or reference values due to the measuring method or measuring conditions.

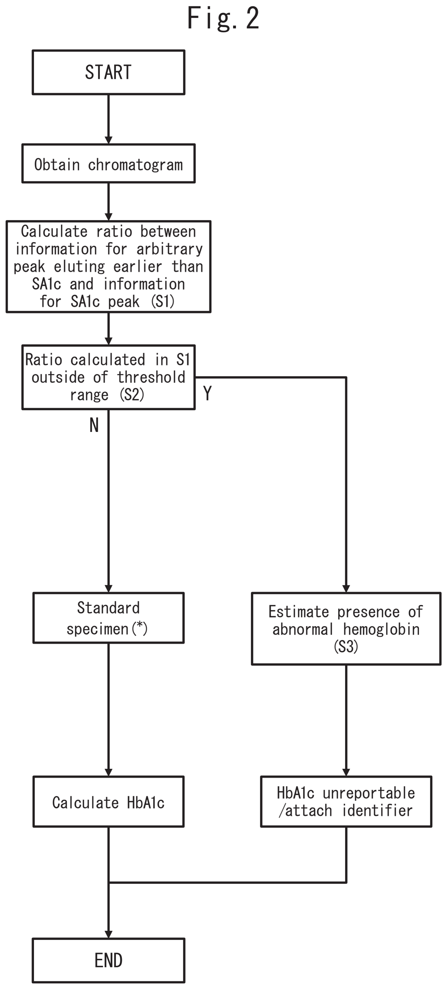

Fig. 2 is a flow chart illustrating the method of the invention according to one aspect. However, the order of steps does not need to be according to the illustrated flow chart, and some steps may be omitted or added.

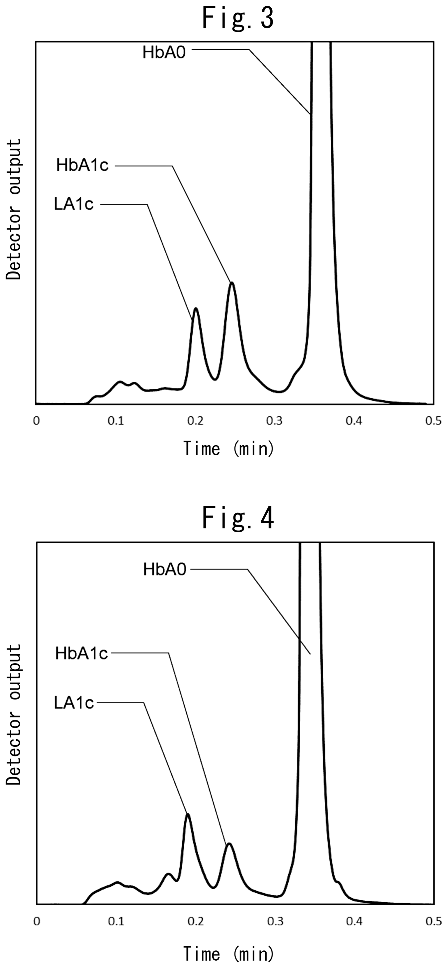

Fig. 3 shows an example of a chromatogram obtained by HbAlc measurement of a common specimen (healthy human-derived specimen). Fig. 4 shows an example of a chromatogram obtained by HbAlc measurement of an HbJ-Baltimore specimen.

In the flow chart shown in Fig. 2, first the ratio between information for an arbitrary peak with earlier elution than SA1c and SA1c peak information is calculated for an obtained chromatogram (S1). An example of a peak with earlier elution than SA1c (corresponding to the second peak) is hemoglobin F (HbF) or LA1c, which is labile HbA1c, and it may also be a peak that includes multiple hemoglobin fractions in a single peak. The peak information to be compared may be, for example, the peak area, peak height or peak area ratio. The peak area ratio is the ratio (percentage) of the area of a peak of interest with respect to the total hemoglobin area (total area), and it may be represented as a ratio or as a percentage (%). The comparison results for the peak information calculated in S1 are compared against the previously recorded predetermined reference threshold range (S2). When the measured value has deviated from the predetermined reference threshold range, it may be estimated that abnormal hemoglobin is highly likely to be present in the specimen (S3). When it is estimated that abnormal hemoglobin is present in the specimen but there is high probability that the measured HbAlc results are not accurate, a course of action may be taken corresponding to the inability to report the measurement results, such as not representing or printing the measurement results for HbA1c, or not transferring the HbAlc results to a high-end host computer. An identifier such as a message or flag may also be appended to the measurement results to indicate the possibility that abnormal hemoglobin may be present.

When the value is within the predetermined reference threshold range in the comparison in S2, on the other hand, it may be judged (estimated) that abnormal hemoglobin such as HbJ-Baltimore is not present. Such specimens may potentially be abnormal Hb specimens in addition to standard specimens, but herein they are listed as "standard specimen(*)" including any abnormal hemoglobin without a characteristic peak, eluting earlier than SA1c.

According to one embodiment of the method the invention,
the second peak is a peak for LA1c,
in step (2), the first peak information is the area ratio for HbAlc and the second peak information is the area ratio for LA1c, and
the ratio of the first peak information and the second peak information in step (3) may be the ratio of the area ratio for LA1c and the area ratio for HbAlc (LA1c%/SA1c%). In this case, the predetermined reference threshold range may be set to be less than 0.50 or less than 0.70. For example, if the LA1c%/SA1c% value exceeds 0.50 or exceeds 0.70, it may be estimated that abnormal hemoglobin is present in the sample being measured.

The present invention will now be described in greater detail by Examples and Comparative Examples, with the understanding that the invention is not limited to the Examples.

### [Examples]

### (Example 1)

For this Example, measurement was carried out in standard short mode with 30 seconds measurement per specimen, and the LA1c peak was used as the peak with earlier elution than SA1c, with the peak area ratio as the compared peak information. The area ratio for SA1c was the value after calibration.

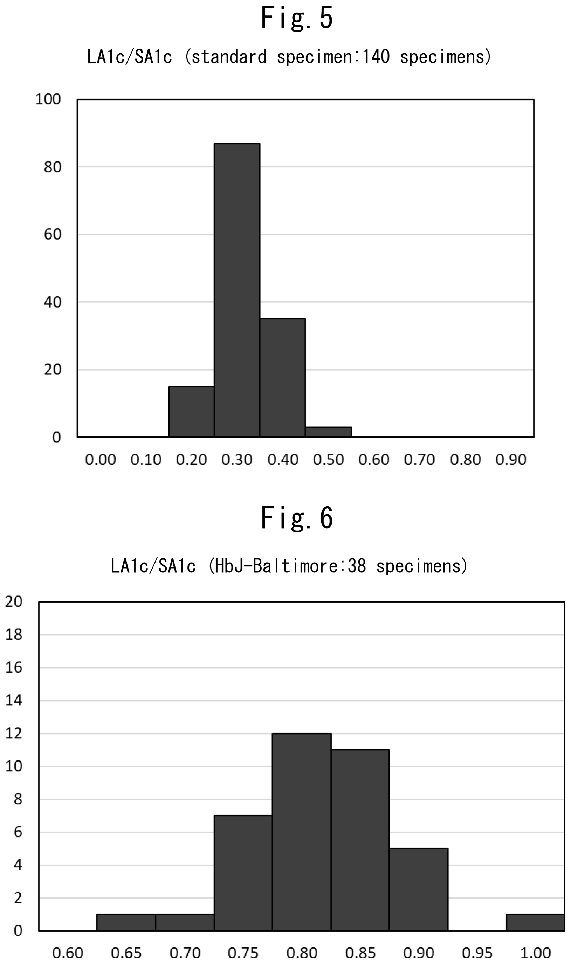

After measuring 140 specimens containing no abnormal hemoglobin, the LA1c%/SA1c% ratio was determined and the values and frequency were confirmed, to obtain the histogram shown in Fig. 5. It was found that for specimens lacking abnormal hemoglobin, the LA1c%/SA1c% ratio was in the range of 0.10 to 0.60, regardless of the HbAlc concentration (SA1c area ratio).

The LA1c%/SA1c% ratio was then determined for a measuring example using 38 specimens estimated to have HbJ-Baltimore, and the values and frequency were confirmed to obtain the histogram shown in Fig. 6. In all of the specimen measurement examples, the ratio was found to be in the range of 0.65 to 1.00.

Based on these results, 0.60 can be set as a predetermined reference threshold for the LA1c%/SA1c% ratio. Thus, when the LA1c%/SA1c% ratio has exceeded this value for a measured specimen, an identifier may be provided that suggests that abnormal hemoglobin might be present, or the HbAlc measurement results for the specimen may be considered to be non-reportable (for example, no display or printing occurs). This can reduce reporting of measurement results that may lead to misdiagnosis, even when measuring specimens with HbJ-Baltimore that affect HbA1c measurement results.

An identifier suggesting that abnormal hemoglobin might be present may be a flag or message attached to the results, or it may be a direct indicator of the abnormal hemoglobin name or the presence of abnormal hemoglobin. Alternatively, the possibility of the presence of abnormal hemoglobin may be indirectly indicated by describing the phenomenon, such as an excessive ratio of LA1c%/SA1c%, for example. When data in the form of a correspondence table are provided, an identifier consisting entirely of numerical values or symbols may be attached.

### (Example 2)

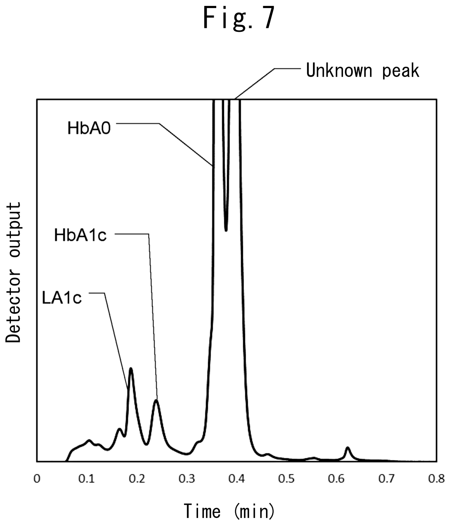

For this Example, HbJ-Baltimore was measured in standard long mode with 50 seconds measurement per specimen. As a result of measuring one specimen for this Example, a chromatogram was obtained as shown in Fig. 7. Calculation of the peak area ratio yielded the results: LA1c%: 4.46% and SA1c%: 5.48%, for an LA1c%/SA1c% ratio of 0.81. These values were within the threshold range of 0.6 to 1.0, suggesting the possibility of HbJ-Baltimore. An unknown peak was also detected after A0. After additional measurement of another specimen for this Example and calculation of the peak area ratio, the result was: LA1c%: 3.12% and SA1c%: 4.05%, for an LA1c%/SA1c% ratio of 0.77. These values were also within the threshold range of 0.6 to 1.0, suggesting the possibility of HbJ-Baltimore. In this specimen as well, an unknown peak was detected after A0.

It was thus demonstrated that the predetermined threshold range set in Example 1 can be used as a common range even for different measuring modes. It was also demonstrated that detection of one or more peaks other than the HbA0 peak which elute later than the SA1c peak, can be added as one condition allowing the presence of HbJ-Baltimore to be estimated.

### REFERENCE SIGNS LIST

1 Analyzing means
2 Cation-exchange liquid chromatography apparatus
10 Eluent first solution
11 Eluent second solution
12 Eluent third solution
13 Degasser
14 Liquid feed pump
15, 16, 17 Solenoid valve
18 Autosampler
19 Analysis column
20 Detector
21 Column oven
22 Control/processing unit
23 Storage unit
24 Display screen (input device)
25 Data storage unit

## Claims

1. A method for analyzing hemoglobin from a blood-derived sample, the method comprising:
(1) a step of identifying a first peak for HbAlc and a different peak (second peak) with earlier elution than the first peak, from a chromatogram representing elution of a plurality of hemoglobin fractions including at least HbAlc and HbA0, obtained by supplying a blood-derived sample to cation-exchange liquid chromatography,
(2) a step of calculating first peak information for the first peak and second peak information for the second peak, wherein the first peak information and the second peak information each consist of the respective peak heights, peak areas or peak area ratios; and
(3) a step of estimating the presence of abnormal hemoglobin in the sample based on comparison between the first peak information and the second peak information and a predetermined reference threshold range.

2. The method according to claim 1, wherein the second peak is a peak for hemoglobin F or LA1c.

3. The method according to claim 1, wherein in step (3), it is estimated that abnormal hemoglobin is present in the sample when the ratio of the first peak information and the second peak information has deviated from the predetermined reference threshold range.

4. The method according to claim 1, wherein in step (3), it is estimated that abnormal hemoglobin is present in the sample when the ratio of the first peak information and the second peak information has deviated from the predetermined reference threshold range, and in the chromatogram, one or more peaks have been detected that elute later than the first peak and are different from the peak for HbA0.

5. The method according to claim 3, wherein when it is estimated that the abnormal hemoglobin is present in the sample, one or more steps selected from among the following steps are carried out:
(a) the response given is that the measurement results for HbAlc for the sample cannot be reported; and
(b) the response given is the measurement results for HbAlc for the sample, with an associated identifier indicating the presence of the abnormal hemoglobin.

6. The method according to claim 1, wherein the abnormal hemoglobin is hemoglobin J-Baltimore.

7. The method according to claim 1, wherein
the second peak is a peak for LA1c,
in step (2), the first peak information is the area ratio for HbAlc and the second peak information is the area ratio for LA1c, and
the ratio of the first peak information and the second peak information in step (3) is the ratio of the area ratio for LAlc and the area ratio for HbAlc (LA1c%/SA1c%).

8. The method according to claim 7, wherein the predetermined reference threshold range is less than 0.50 or less than 0.70.

9. A hemoglobin analysis system in which the method according to claim 1 is carried out.

10. The hemoglobin analysis system according to claim 9, which comprises:
a cation-exchange liquid chromatography apparatus, and
analyzing means for analyzing chromatogram data obtained by the cation-exchange liquid chromatography apparatus.
